# EUROPEAN PATENT APPLICATION

(11) **EP 2 409 654 A2**
(43) Date of publication of application: **25.01.2012**
(21) Application number: 11250663.9
(22) Date of filing: 18.07.2011
(51) Int. Cl.: A61B 17/115, A61B 17/072, A61B 17/00

(54) **Anvil assembly for surgical stapling device**

(30) Priority: 19.07.2010 US 365403 P; 09.06.2011 US 156645
(71) Applicant: Tyco Healthcare Group LP, New Haven, CT 06511 (US)
(72) Inventor: Milliman, Keith L., Bethel, CT 06801 (US); Hessler, Thomas, Bethel, CT 06801 (US)
(74) Representative: Soames, Candida Jane

(57) **Abstract**

An anvil assembly for use with a surgical fastener applying apparatus. The anvil assembly includes an anvil center rod (40) and anvil head (42) that is secured to the distal end portion of the anvil center rod. The anvil center rod and the anvil head include corresponding mating structures that are configured and dimensioned to facilitate connection of the anvil center rod and the anvil head such that relative movement between the anvil center rod and the anvil head is substantially inhibited. The anvil center rod and the anvil head include corresponding mating structures that are non-circular in configuration. A method of assembling a surgical anvil assembly is also disclosed.

## Description

### BACKGROUND

This application claims priority from provisional application serial no. 61/365,403, filed July 19, 2010, the entire contents of which are incorporated herein by reference.

### 1. Technical Field

The present disclosure relates to surgical fastener applying apparatus, and more particularly, to an anvil assembly for use therewith, as well as methods of manufacturing the same.

### 2. Background of Related Art

Many varieties of surgical fastener applying apparatus are known in the art, some of which are specifically adapted for use in various surgical procedures, such as end-to-end anastomosis, circular end-to-end anastomosis, open gastrointestinal anastomosis, endoscopic gastrointestinal anastomosis, and transverse anastomosis. Suitable examples of fastener applying apparatus that may be used during such procedures are described in U.S. Pat. Nos. 5,865,361; 5,915,616; 5,964,394; and 6,202,914.

Surgical fastener applying apparatus for use in performing circular anastomosis of hollow tissue organs generally include an anvil assembly incorporating an anvil head assembly and a center rod assembly. The anvil head assembly includes, among other components, an anvil head and an anvil plate with pockets formed therein that are configured and dimensioned to receive and form surgical fasteners in order to join together adjacent sections of the patient's tissue. The fastener pockets are typically arranged into one or more rows positioned on opposite sides of, or about, a cutting member or a corresponding channel adapted for the receipt of the cutting member.

When the surgical fasteners are applied to tissue, proper formation is desirable for many different reasons, including the minimization of bleeding and the effectuation of hemostasis. In order to ensure the accurate and consistent formation of surgical fasteners, considerable research and development has been conducted in the areas of forming and driving structures, and strict manufacturing tolerances have been implemented. For example, anvil assemblies such as those described in U.S. Pat. Nos. 5,173,133 and 5,480,089 have been developed with specific coatings and/or structure, and fastener cartridges such as those described in U.S. Pat. No. 4,978,049 include driver structure that is configured and dimensioned to balance forces encountered during staple formation.

Additionally, simplicity of design remains a constant goal in the interests of decreasing assembly time and reducing manufacturing costs.

### SUMMARY

In one aspect of the present disclosure, a surgical anvil assembly is provided that includes an anvil center rod and an anvil head. The anvil center rod has proximal and distal end portions and extends along a longitudinal axis. The anvil head is secured to the distal end portion of the anvil center rod.

The anvil center rod and the anvil head include corresponding mating structures that are configured and dimensioned to facilitate connection of the anvil center rod and the anvil head such that relative movement between the anvil center rod and the anvil head is substantially inhibited. The corresponding mating structures included on the anvil center rod and the anvil head are each non-circular in configuration.

The mating structure on the anvil center rod preferably includes a first arcuate portion and a first linear portion, and the mating structure on the anvil head preferably includes a second arcuate portion and a second linear portion, wherein the first arcuate portion is configured and dimensioned for engagement with the second arcuate portion and the first linear portion is configured and dimensioned for engagement with the second linear portion.

The anvil center rod and the anvil head preferably connect in an interference fit arrangement, and in one embodiment of the present disclosure, the anvil head includes a bearing member defining a bore that is configured and dimensioned to receive the distal end of the anvil center rod.

The distal end of the anvil center rod may include a flange that projects radially outward relative to the longitudinal axis, and a bearing member of the anvil head may be adapted for deformation to thereby define a shoulder that is configured and dimensioned for engagement with the flange.

The anvil assembly may also include a cap member that is configured and dimensioned for engagement with the anvil head. In such embodiments, the anvil head and the cap member may be configured and dimensioned for engagement in snap-fit relation. For example, the anvil head may include a distal surface with a flange, and the cap member may include an inner cavity with one or more detents positioned therein that are configured, dimensioned, and adapted for displacement by the flange upon advancement of the flange through the inner cavity. In order to facilitate displacement of the one or more detents by the flange formed on the distal surface of the anvil head, the cap member may be at least partially formed from a resilient material.

In another aspect of the present disclosure, a surgical fastener applying apparatus is provided including an elongated body portion with proximal and distal end portions, a shell assembly that is positioned at the distal end portion of the elongated body portion and configured and dimensioned to retain a plurality of surgical fasteners, and an anvil assembly having an anvil center rod extending along a longitudinal axis and having a proximal end portion and a distal end portion and an anvil head secured to the distal end portion of the anvil center rod. The anvil center rod and the anvil head include corresponding mating structures configured and dimensioned to facilitate connection of the anvil center rod and the anvil head such that relative movement between the anvil center rod and the anvil head is substantially inhibited, wherein the corresponding mating structures included on the anvil center rod and the anvil head are each non-circular in configuration.

In another aspect, a method of manufacturing an anvil assembly for use with a surgical fastener applying apparatus is disclosed. The method comprises the steps of providing an anvil head defining a longitudinal axis, providing an anvil plate including an outer side wall, positioning the anvil head and the anvil plate in contacting relation, and roll forming a portion of the anvil head towards the anvil plate to fixedly secure the anvil head and the anvil plate together such that the anvil assembly includes a substantially uniform proximal-most surface.

In yet another aspect of the present disclosure, a method of assembling a surgical anvil assembly is provided that includes the steps of (i) inserting a distal end of a center rod into an anvil head until a flange included at the distal end of the center rod is positioned within a bearing member defining a bore formed in the anvil head; (ii) deforming a portion of the bearing member radially inward to define a shoulder that is configured and dimensioned for engagement with the flange; and (iii) attaching a cap member to a distal surface of the anvil head.

It is envisioned that the step of inserting a distal end of the center rod into the anvil head may include aligning corresponding non-circular mating structures formed on the anvil center rod and the anvil head such that an interference fit is created between the anvil center rod and the anvil head upon assembly in order to substantially inhibit relative movement therebetween.

These and other features of the presently disclosed anvil assembly, surgical fastener applying apparatus, and corresponding methods of assembly disclosed herein will become more readily apparent to those skilled in the art through reference to the detailed description of the various embodiments provided below.

### BRIEF DESCRIPTION OF THE DRAWINGS

Various embodiments of the presently disclosed anvil assembly and surgical fastener applying apparatus will be described herein with reference to the accompanying drawings, wherein:

FIG. 1 is a top, perspective view of a surgical fastener applying apparatus including an anvil assembly in accordance with the principles of the present disclosure;

FIG. 2 is a front, perspective view of an exemplary surgical fastener that may be utilized with the surgical fastener applying apparatus seen in FIG. 1;

FIG. 3 is a side, perspective view illustrating the presently disclosed anvil assembly;

FIG. 4 is a side, perspective view of the anvil assembly of FIG. 3 with parts separated;

FIG. 5A is a top, perspective view of the anvil assembly of FIG. 3 shown with the anvil center rod assembly separated from the anvil head assembly;

FIG. 5B is a top, perspective view of an alternative embodiment of the anvil assembly shown with the anvil center rod assembly separated from the anvil head assembly;

FIG. 6 is a top, perspective view of the anvil assembly of FIG. 3 following connection of the anvil center rod assembly and the anvil head assembly;

FIG. 7 is a longitudinal, cross-sectional view of the anvil assembly of FIG. 3;

FIG. 8 is an enlarged view of the area of detail indicated in FIG. 7;

FIG. 9 is a transverse, cross-sectional view of the anvil head assembly taken through line 9-9 in FIG. 4;

FIG. 10 is a transverse, cross-sectional view of the anvil center rod assembly taken through line 10-10 in FIG. 4;

FIG. 11 is a bottom, cross-sectional view of the anvil assembly taken through line 11-11 in FIG. 3 shown in perspective; and

FIG. 12 is a bottom, cross-sectional view of the anvil assembly taken through line 11-11 in FIG. 3.

### DESCRIPTION OF VARIOUS EMBODIMENTS

Various exemplary embodiments of the presently disclosed anvil assembly and surgical fastener applying apparatus, as well as the associated methods of manufacturing the same, will now be described in detail with reference to the drawings wherein like references characters identify similar or identical elements. In the drawings, and in the following description, the term "proximal" will refer to the end of the anvil assembly, surgical fastener applying apparatus, or component thereof, that is closer to the operator during proper use, while the term "distal" will refer to the end that is further from the operator, as is traditional and conventional in the art. In addition, the term "surgical fastener" should be understood to include any substantially rigid structure formed of a biocompatible material that is suitable for the intended purpose of joining together adjacent tissue portions, including but not being limited to surgical staples, clips, and the like.

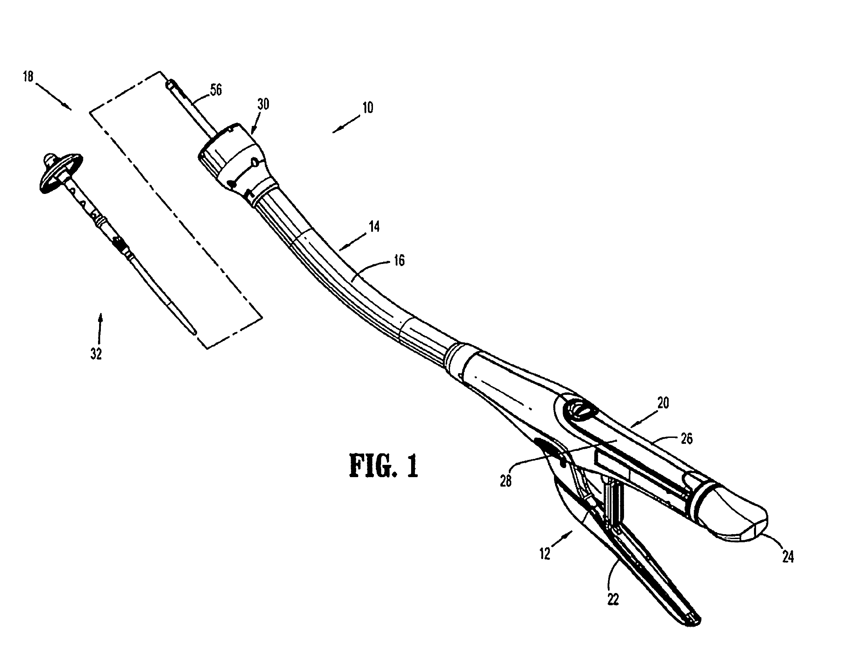

FIG. 1 illustrates a surgical fastener applying apparatus, referred to generally by the reference character 10. Briefly, the surgical fastener applying apparatus 10 includes a handle assembly 12, an elongated central body portion 14 with an elongated outer tube 16, and a distal head portion 18. In alternative embodiments of the surgical fastener applying apparatus 10, the length and/or configuration of the central body portion 14 may be altered or varied to suit the requirements of the particular surgical procedure in which the surgical fastener applying apparatus may be employed. For example, the central body portion 14 may be either substantially straight, e.g., when intended for use in a procedure for the treatment of hemorrhoids, or alternatively, the central body portion may be curved. The transverse dimensions of the body portion 14 and/or the head portion 18 may also be varied to suit a particular surgical procedure.

The handle assembly 12 includes a stationary handle 20, a firing trigger 22, and a rotatable approximation knob 24. In the embodiment of the surgical fastener applying apparatus 10 shown in FIG. 1, the stationary handle 20 includes a pair of handle sections 26, 28, e.g., formed from polycarbonate, which together define a housing for additional internal components of the handle assembly 12. The handle sections 26, 28 may be secured together by sonic welding, or any other known securement technique, such as through the use of screws, adhesives, snap-fit connectors, rivets, or the like.

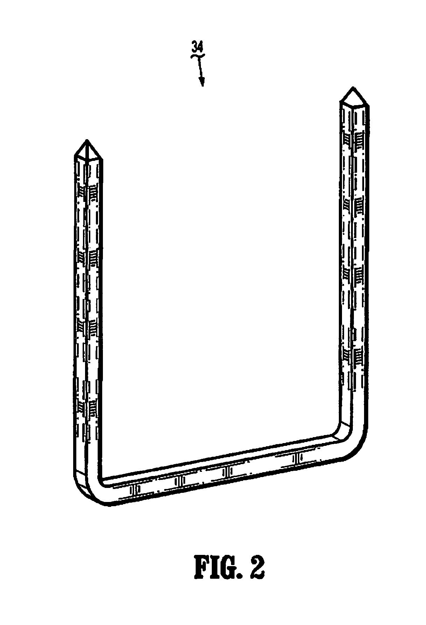

Referring still to FIG. 1, the head portion 18 includes a shell assembly 30, and an anvil assembly 32. The shell assembly 30 is configured, dimensioned, and adapted to accommodate a plurality of surgical fasteners 34, an example of which can be seen in FIG. 2, that are used to attach adjacent portions of a patient's tissue. The shell assembly 30 includes additional components, and performs additional functions, each of which is discussed in U.S. Patent Application Publication No. 20080190991, U.S. Patent Application Serial No. 12/550,443, filed August 31, 2009, U.S. Patent Application Serial No. 12/348,953, filed January 6, 2009 and U.S. Patent Application Serial No. 12/688,870, filed January 16, 2010 the entire contents of each of these applications being incorporated by reference herein. These applications also describe the operations for approximating the anvil assembly and fining the fasteners which are applicable to this disclosure.

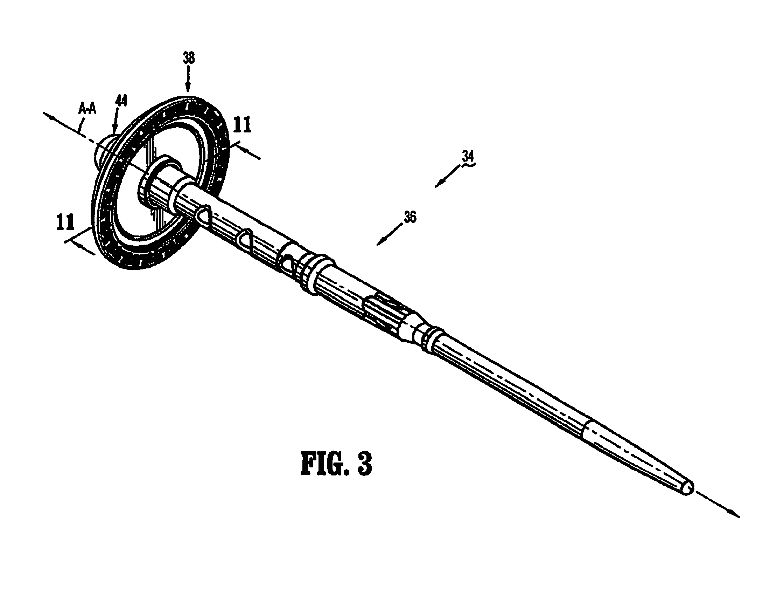

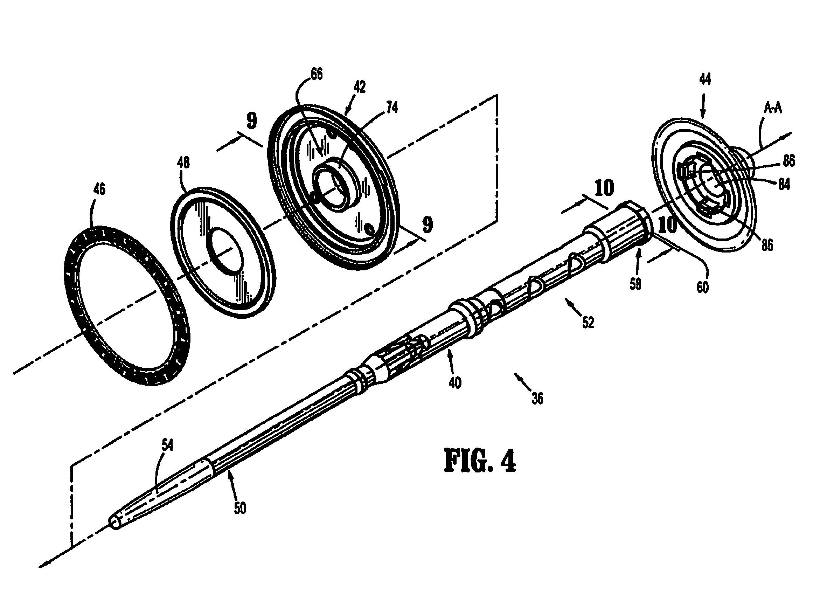

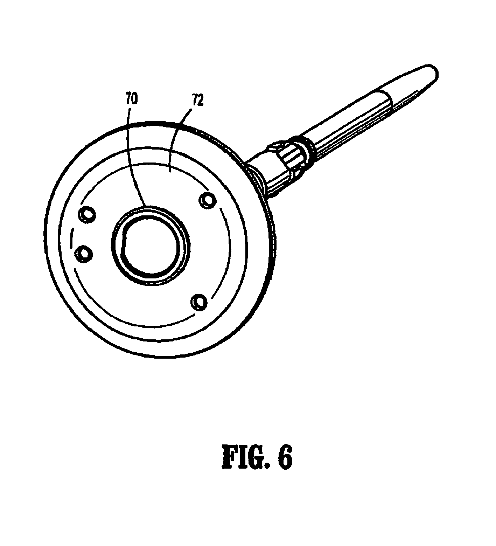

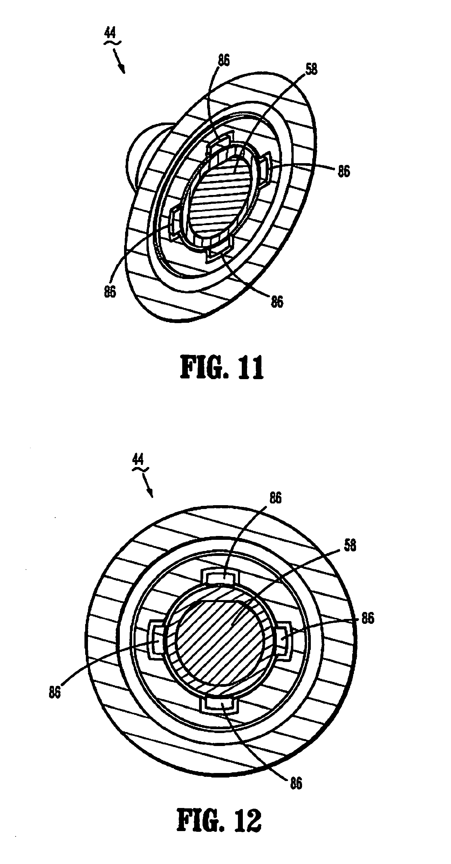

Referring now to FIGS. 3-12, the anvil assembly 32 will be discussed. The anvil assembly 32 extends along a longitudinal axis "A-A," and includes an anvil center rod assembly 36 and an anvil head assembly 38. As best seen in FIGS. 3 and 4, the anvil center rod assembly 36 includes a center rod 40, and the anvil head assembly 34 includes an anvil head 42, a cap member 44, an anvil plate 46, and a cutting ring 48. To attach the anvil head 42 and anvil plate 46, the edge of head 42 can be roll formed, thereby providing a smooth transition at the edge, i.e. a substantially flush edge. That is, this roll forming process secures a stamped anvil plate to the anvil head 42, resulting in a "lipless anvil" due to the resulting substantially uniform proximal most surface.

The anvil head assembly 34 and the anvil center rod assembly 36 include corresponding mating structures that are configured and dimensioned for connection such that relative movement between the anvil head assembly 38 and the anvil center rod assembly 36 is substantially inhibited, if not entirely prevented. The present disclosure is directed primarily to the specific structures of the anvil head 40 and the anvil center rod assembly 36 that facilitate connection in this manner, and accordingly, only the particular structures relevant to connection of the anvil head assembly 38 and the center rod assembly 36 will be discussed in further detail hereinbelow. However, a more detailed discussion of the anvil center rod assembly 36 and the anvil head assembly 34, including the features and elements thereof, is provided in U.S. Patent Application Publication No. 20080190991, and U.S. Patent Application Serial No. 12/688,870, each of which was mentioned and incorporated by reference above.

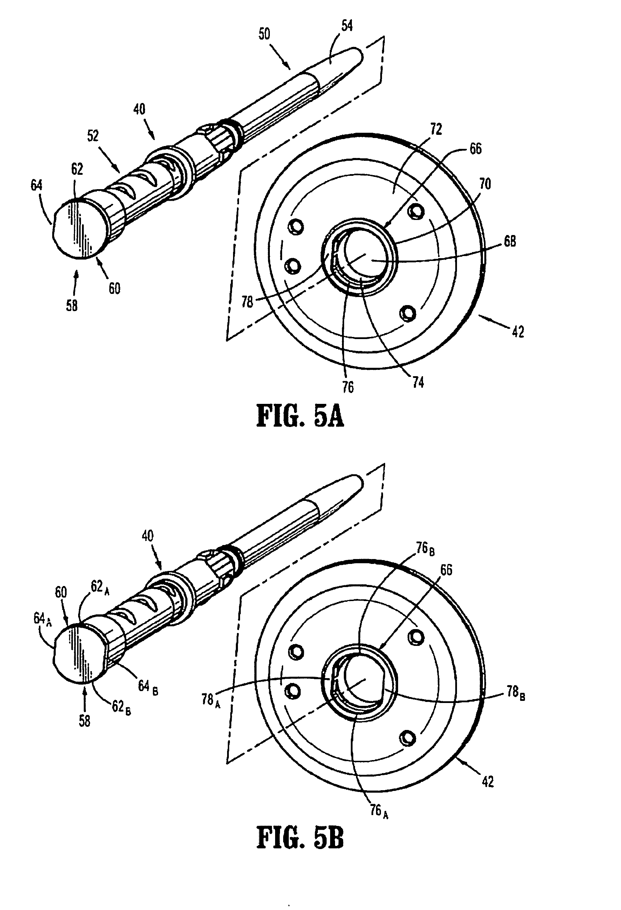

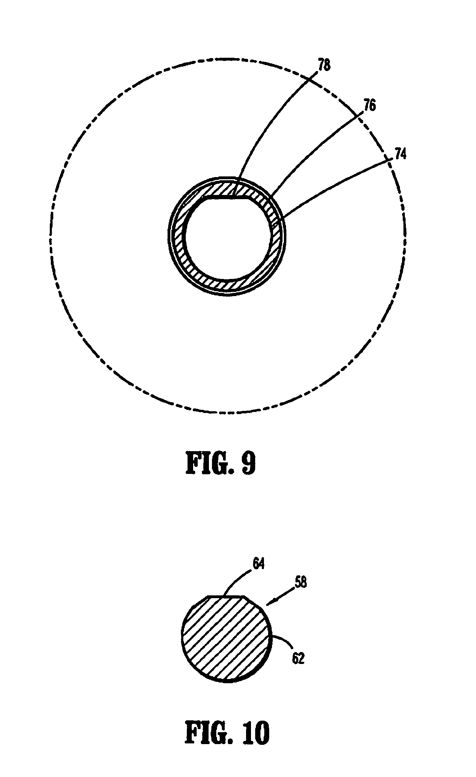

With particular reference now to FIGS. 4 and 5A, the center rod 40 of the anvil center rod assembly 36 includes a proximal portion 50, a central portion including an annular rib, and a distal portion 52. The proximal portion 50 of the center rod 40 includes an atraumatically tapered end 54 that is configured and dimensioned for releasable engagement with an anvil retainer component 56 (FIG. 1) of the shell assembly 30, and the distal portion 52 of the center rod 40 includes a distal end 58 with a generally cylindrical configuration that defines a flange 60 extending radially outward from the longitudinal axis "A-A." In the particular embodiment of the center rod 40 illustrated in the accompanying drawings, the flange 60 at the distal end 58 of the center rod 40 includes an arcuate portion 62 (FIGS. 5A, 10), and a flat 64 with a substantially linear configuration, thereby attributing an approximate "D-shape" to the distal end 58 of the center rod 40.

Referring now to FIGS. 4 and 5A, the anvil head 42 may be formed from any suitable biocompatible material, e.g., stainless steel, and includes a hollow bearing member 66 defining a bore 68 that is configured and dimensioned to receive the distal end 58 of the center rod 40. A flange 70 is positioned about the bore 68 on a distal surface 72 of the anvil head 42. The configurations and dimensions of the bore 68 formed in the anvil head 42 correspond to those of the distal end 58 of the center rod 40 such that when the distal end 58 of the center rod 40 is positioned within the bore 68, an interference fit is created. Consequently, in the particular embodiment of the anvil head 42 illustrated throughout the drawings, the bearing member 66 includes a generally cylindrical configuration with a side wall 74 (FIGS. 4, 5A, 9) having an arcuate portion 76, and a substantially linear flat 78.

Although the flange 60 at the distal end 58 of the center rod 40, and the anvil head 42 are each illustrated as including a single flat, i.e., flats 64 and 78, respectively, in alternate embodiments additional flats may be included. For example, in the embodiment illustrated in FIG. 5B, the flange 60 at the distal end 58 of the center rod 40 includes a pair of opposing arcuate portions 62_{A}*,* 62_{B} that are connected by a pair of opposing substantially linear flats 64_{A}, 64_{B}, and the bearing member 66 of the anvil head 42 includes a pair of opposing arcuate portions 76_{A}, 76_{B} that are connected by a pair of opposing substantially linear flats 78_{A}, 78_{B}. These regions are configured and dimensioned to provide an interference fit of the center rod 40 and anvil head 42.

The interference press fit established between the distal end 58 of the center rod 40 and the bore 68 formed in the anvil head 42 of both embodiments (FIG. 5A and FIG. 5B) inhibits, if not completely prevents, relative movement between the anvil center rod assembly 36 and the anvil head assembly 38, as indicated above. Inhibiting relative movement between the anvil center rod assembly 36 and the anvil head assembly 38 facilitates proper formation of the surgical fasteners within the patient's tissue upon firing of the surgical fastener applying apparatus 10 (FIG. 1). Additionally, by employing an interference fit between the center rod 40 and the anvil head 42, the overall number of components in the head portion 18 (FIG. 1) can be minimized to thereby minimize manufacturing costs. Moreover, the flats facilitate alignment of the anvil head 42 with the splines on the center rod 40 which in turn facilitates alignment of the anvil pockets with the fasteners of the shell assembly.

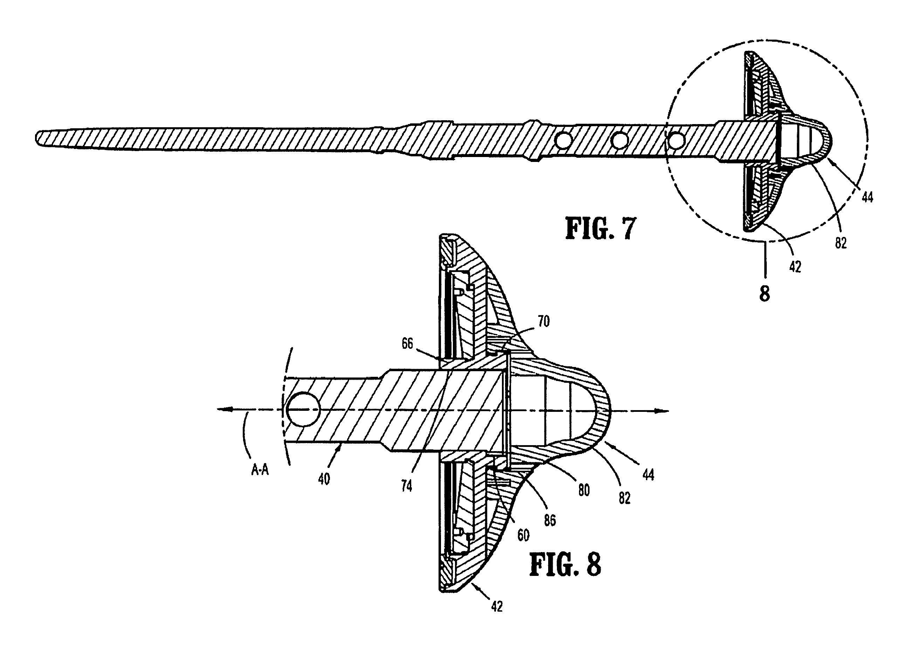

With reference to FIGS. 4-6, 9, and 10, during assembly of the head portion 18, the arcuate portion 62 and the flat 64 included on the distal end 58 of the center rod 40 are aligned with the arcuate portion 76 and the flat 78 of the bore 68 formed in anvil head 42, and the distal end 58 of the center rod 40 is inserted into the bore 68. (For assembly of the embodiment of FIG. 5A, flats 64A and arcuate portions 62_{A}, 62_{B} are aligned with flats 78_{A}, 78_{B} and arcuate portions 76_{A}, 76_{B}). Thereafter, the anvil head 42 and the center rod 40 are secured together. For example, the anvil head 42 may be welded to the center rod 40, or alternatively, the bearing member 66 can be deformed, e.g., by staking or swaging, such that the side wall 74 is moved radially inward towards the longitudinal axis "A-A" to define a shoulder 80 (FIG. 8). As can be appreciated through reference to FIG. 8, in the illustrated embodiment, the shoulder 80 engages the flange 60 to further enhance the connection between the center rod 40 and the anvil head 42. The anvil assembly of Figure 5B can be assembled in a similar fashion.

With reference now to FIGS. 4, 7, 8, 11, and 12, the cap member 44 will be discussed. The cap member 44 is configured and dimensioned for engagement with the anvil head 42, and may be formed from any suitable biocompatible material, and is preferably formed of polymeric materials. The cap member 44 includes an outer surface 82 with a smooth configuration that facilitates atraumatic insertion of the head portion 18 (FIG. 1) into the patient, and an inner cavity 84 (FIG. 4) that is configured and dimensioned to receive the flange 70 (FIG. 5A) that is positioned about the bore 68 formed in the anvil head 42. The cap member 44 further includes one or more detents 86 that extend radially inward into the inner cavity 84 (FIG. 4). The detent(s) 86 are configured and dimensioned for engagement with the flange 70 of anvil head 42 such that the cap member 44 can be snap-fit to the anvil head 42. More specifically, during assembly of the head portion 18, upon engagement of the detent(s) 86 with the flange 70, the detent(s) 86 are deflected radially outward, i.e., away from the longitudinal axis "A-A," such that the flange 70 can be further advanced through the cap member 44 until the flange 70 is positioned distally of the detent(s) 86, as shown in FIG. 8. Thereafter, the detent(s) 86 move towards their original position to thereby secure the flange 70 within the inner cavity 84.

The above description, disclosure, and figures should not be construed as limiting, but merely as exemplary of particular embodiments. It is to be understood, therefore, that the disclosure is not limited to the precise embodiments described, and that various other changes and modifications may be effected by one skilled in the art without departing from the scope or spirit of the present disclosure. Additionally, persons skilled in the art will appreciate that the features illustrated or described in connection with one embodiment may be combined with those of another, and that such modifications and variations are also intended to be included within the scope of the present disclosure.
The invention may be described by reference to the following numbered paragraphs:-
1. An anvil assembly for a surgical stapler comprising:
   an anvil center rod extending along a longitudinal axis, and having a proximal end portion and a distal end portion; and
   an anvil head secured to the distal end portion of the anvil center rod, the anvil center rod and the anvil head including corresponding mating structures configured and dimensioned to facilitate connection of the anvil center rod and the anvil head such that relative movement between the anvil center rod and the anvil head is substantially inhibited, wherein the corresponding mating structures included on the anvil center rod and the anvil head are each non-circular in configuration.
2. The anvil assembly of paragraph 1, wherein the anvil center rod includes a first arcuate portion and a first linear portion, and the anvil head includes a second arcuate portion and a second linear portion, the first arcuate portion being configured and dimensioned for engagement with the second arcuate portion, and the first linear portion being configured and dimensioned for engagement with the second linear portion.
3. The anvil assembly of paragraph 1, wherein the anvil center rod and the anvil head are configured and dimensioned for connection in an interference fit arrangement.
4. The anvil assembly of paragraph 3, wherein the anvil head includes a bearing member defining a bore configured and dimensioned to receive the distal end of the anvil center rod.
5. The anvil assembly of paragraph 4, wherein the distal end of the anvil center rod includes a flange projecting radially outward relative to the longitudinal axis, and the bearing member of the anvil head is adapted for deformation to thereby define a shoulder configured and dimensioned for engagement with the flange to further secure the anvil center rod and the anvil head.
6. The anvil assembly of paragraph 1, further including a cap member configured and dimensioned for engagement with the anvil head.
7. The anvil assembly of paragraph 6, wherein the anvil head and the cap member are configured and dimensioned for engagement in snap-fit relation.
8. The anvil assembly of paragraph 7, wherein the anvil head includes a distal surface with a flange, and the cap member includes an inner cavity with one or more detents positioned therein, the detents being configured, dimensioned, and adapted for displacement by the flange formed on the distal surface of the anvil head upon advancement of the flange through the inner cavity.
9. The anvil assembly of paragraph 8, wherein the cap member is at least partially formed from a resilient material to facilitate displacement of the one or more detents by the flange formed on the distal surface of the anvil head.
10. A surgical fastener applying apparatus, comprising:
   an elongated body portion having proximal and distal ends;
   a shell assembly positioned at the distal end of the elongated body portion, the shell assembly being configured and dimensioned to retain a plurality of surgical fasteners; and
   an anvil assembly including:
      an anvil center rod extending along a longitudinal axis, and having a proximal end portion and a distal end portion; and
      an anvil head secured to the distal end portion of the anvil center rod, the anvil center rod and the anvil head including corresponding mating structures configured and dimensioned to facilitate connection of the anvil center rod and the anvil head such that relative movement between the anvil center rod and the anvil head is substantially inhibited, wherein the corresponding mating structures included on the anvil center rod and the anvil head are each non-circular in configuration.
11. The surgical fastener applying apparatus of paragraph 10, wherein the anvil center rod includes a first arcuate portion and a first linear portion, and the anvil head includes a second arcuate portion and a second linear portion, the first arcuate portion being configured and dimensioned for engagement with the second arcuate portion, and the first linear portion being configured and dimensioned for engagement with the second linear portion.
12. The surgical fastener applying apparatus of paragraph 10, wherein the anvil center rod and the anvil head are configured and dimensioned for connection in an interference fit arrangement.
13. The surgical fastener applying apparatus of paragraph 12, wherein the anvil head includes a bearing member defining a bore configured and dimensioned to receive the distal end of the anvil center rod.
14. The surgical fastener applying apparatus of paragraph 13, wherein the distal end of the anvil center rod includes a flange projecting radially outward relative to the longitudinal axis, and the bearing member of the anvil head is adapted for deformation to thereby define a shoulder configured and dimensioned for engagement with the flange to further secure the anvil center rod and the anvil head.
15. The surgical fastener applying apparatus of paragraph 10, further including a cap member configured and dimensioned for engagement with the anvil head.
16. The surgical fastener applying apparatus of paragraph 15, wherein the anvil head and the cap member are configured and dimensioned for engagement in snap-fit relation.
17. The surgical fastener applying apparatus of paragraph 16, wherein the anvil head includes a distal surface with a flange, and the cap member includes an inner cavity with one or more detents positioned therein, the detents being configured, dimensioned, and adapted for displacement by the flange formed on the distal surface of the anvil head upon advancement of the flange through the inner cavity, the cap member at least partially formed from a resilient material to facilitate displacement of the one or more detents by the flange formed on the distal surface of the anvil head.
18. A method of manufacturing an anvil assembly for use with a surgical fastener applying apparatus, the method comprising the steps of:
   providing an anvil head defining a longitudinal axis;
   providing an anvil plate including an outer side wall;
   positioning the anvil head and the anvil plate in contacting relation; and
   roll forming a portion of the anvil head towards the anvil plate to fixedly secure the anvil head and the anvil plate together such that the anvil assembly includes a substantially uniform proximal-most surface.
19. A method of assembling a surgical anvil assembly comprising the steps of:
   inserting a distal end of a center rod into an anvil head until a flange included at the distal end of the center rod is positioned within a bearing member defining a bore formed in the anvil head;
   deforming a portion of the bearing member radially inward to define a shoulder configured and dimensioned for engagement with the flange; and
   attaching a cap member to a distal surface of the anvil head.
20. The method of paragraph 19, wherein the step of inserting a distal end of the center rod into the anvil head includes aligning corresponding non-circular mating structures formed on the anvil center rod and the anvil head such that an interference fit is created between the anvil center rod and the anvil head upon assembly to substantially inhibit relative movement therebetween.

## Claims

1. An anvil assembly for a surgical stapler comprising:
an anvil center rod extending along a longitudinal axis, and having a proximal end portion and a distal end portion; and
an anvil head secured to the distal end portion of the anvil center rod, the anvil center rod and the anvil head including corresponding mating structures configured and dimensioned to facilitate connection of the anvil center rod and the anvil head such that relative movement between the anvil center rod and the anvil head is substantially inhibited, wherein the corresponding mating structures included on the anvil center rod and the anvil head are each non-circular in configuration.

2. The anvil assembly of claim 1, wherein the anvil center rod includes a first arcuate portion and a first linear portion, and the anvil head includes a second arcuate portion and a second linear portion, the first arcuate portion being configured and dimensioned for engagement with the second arcuate portion, and the first linear portion being configured and dimensioned for engagement with the second linear portion.

3. The anvil assembly of claims 1 or 2, wherein the anvil center rod and the anvil head are configured and dimensioned for connection in an interference fit arrangement.

4. The anvil assembly of claim 3, wherein the anvil head includes a bearing member defining a bore configured and dimensioned to receive the distal end of the anvil center rod.

5. The anvil assembly of claim 4, wherein the distal end of the anvil center rod includes a flange projecting radially outward relative to the longitudinal axis, and the bearing member of the anvil head is adapted for deformation to thereby define a shoulder configured and dimensioned for engagement with the flange to further secure the anvil center rod and the anvil head.

6. The anvil assembly of any of claims 1-5, further including a cap member configured and dimensioned for engagement with the anvil head.

7. The anvil assembly of claim 6, wherein the anvil head and the cap member are configured and dimensioned for engagement in snap-fit relation.

8. The anvil assembly of claim 7, wherein the anvil head includes a distal surface with a flange, and the cap member includes an inner cavity with one or more detents positioned therein, the detents being configured, dimensioned, and adapted for displacement by the flange formed on the distal surface of the anvil head upon advancement of the flange through the inner cavity.

9. The anvil assembly of any of claims 6, 7 or 8, wherein the cap member is at least partially formed from a resilient material to facilitate displacement of the one or more detents by the flange formed on the distal surface of the anvil head.

10. The anvil assembly of any of claims 1-8 in combination with a surgical fastener applying apparatus, comprising:
an elongated body portion having proximal and distal ends; and
a shell assembly positioned at the distal end of the elongated body portion, the shell assembly being configured and dimensioned to retain a plurality of surgical fasteners; and

11. A method of manufacturing an anvil assembly for use with a surgical fastener applying apparatus, the method comprising the steps of:
providing an anvil head defining a longitudinal axis;
providing an anvil plate including an outer side wall;
positioning the anvil head and the anvil plate in contacting relation; and
roll forming a portion of the anvil head towards the anvil plate to fixedly secure the anvil head and the anvil plate together such that the anvil assembly includes a substantially uniform proximal-most surface.

12. The method of claim 11, further comprising the step of attaching a cap member to a distal surface of the anvil head.

13. The method of claim 12, wherein the anvil had and cap member engage in a snap-fit relation.

14. A method of assembling a surgical anvil assembly comprising the steps of:
inserting a distal end of a center rod into an anvil head until a flange included at the distal end of the center rod is positioned within a bearing member defining a bore formed in the anvil head;
deforming a portion of the bearing member radially inward to define a shoulder configured and dimensioned for engagement with the flange; and
attaching a cap member to a distal surface of the anvil head.

15. The method of claim 14, wherein the step of inserting a distal end of the center rod into the anvil head includes aligning corresponding non-circular mating structures formed on the anvil center rod and the anvil head such that an interference fit is created between the anvil center rod and the anvil head upon assembly to substantially inhibit relative movement therebetween.
